# EUROPEAN PATENT APPLICATION

(11) **EP 1 333 087 A1**
(43) Date of publication of application: **06.08.2003**
(21) Application number: 02075490.9
(22) Date of filing: 05.02.2002
(51) Int. Cl.: C12N 9/96

(54) **Crosslinked enzyme aggregates and crosslinking agent therefore**

(71) Applicant: Avantium International B.V., 1014 BV Amsterdam (NL)
(72) Inventor: Cao, Linqiu, 2624 PP Delft (NL); Elzinga, Jeoffrey, 2614 AK Delft (NL)
(74) Representative: Wittop Koning, Tom Hugo

(57) **Abstract**

Described is a method for the preparation of crosslinked enzyme aggregates, comprising the steps of:
A - providing a plurality of enzyme molecules,
B - aggregating the enzymes in a liquid medium, comprising a precipitating agent,
C - crosslinking the aggregated enzymes to one another by providing a crosslinking agent in the liquid medium,
wherein the crosslinking agent being prepared by combining a first and a second compound each having at least two reactive groups, the reactive groups of the first compound being primary amino groups, the reactive groups of the second compound being aldehyde groups.

## Description

The present invention relates to a method for the preparation of crosslinked enzyme aggregates, comprising the steps of:
A - providing a plurality of enzyme molecules,
B - aggregating the enzymes in a liquid medium, comprising a precipitating agent.
C - crosslinking the aggregated enzymes to one another by providing a crosslinking agent in the liquid medium, to a crosslinked enzyme aggregate, obtainable by the said method and to a crosslinking agent, useable for the preparation of the said crosslinked enzyme aggregates.

A method for the preparation of crosslinked enzyme aggregates (further also abbreviated as CLEAs) of the above-mentioned type is known from EP-A-1 088 887, herein incorporated by reference. Crosslinked enzyme aggregates can be regarded as self-supported immobilised enzymes, having potentially broad applicability (L. Cao et al., Org. Lett., 2000, 2, 1301-1364 and L. Cao et al., J. Mol. Cat. B: Enzymatic, 2001, 11, 665-670).

Crosslinked enzyme aggregates can advantageously be used as industrial biocatalysts and for analytic and multiple other research purposes. In the said fields, enzymes, or biocatalysts, are often immobilised to a solid carrier, leading to a so called "dilution of activity", because usually, the enzyme only constitutes 5% of the weight of the immobilised mass; 95% or more thereof is constituted by the non-catalytic material.

CLEAs can be used in a plurality of biochemical processes on industrial or laboratory scale, for the preparation of compounds through an enzymatic preparation process. Non-limiting examples thereof are the preparation of β-lactam antibiotics, amino acids, asparatame and structured lipids (Soumanou, M.M., Bornscheuer, U.T., Schmid, U. und Schmid, R.D., Crucial role of support and water activity on the lipase-catalyzed synthesis of structured triglycerides, Biocatalysis Biotransformations 16 (1999) 443-459).

Further, CLEA's can be used for e.g. determining the exact activity of an enzyme. In that case, CLEAs can be used to "freeze" the activity of the enzyme. The performance of the enzyme can be directly related to the activity as a free enzyme as the CLEA preparation allows an easy handling and measurement of activity and easy dosing.

Due to the great range of applications and very different biological sources for enzymes, there are numerous factors to consider when weighing the enzyme assays provided by a manufacturer. This may e.q. be necessary when the enzymes are so powerful, even in small amounts, that the minutest variations in dosage or dilution can wreak havoc. For example, in baked products, it is possible for alpha-amylase enzyme activity to continue even after baking, creating a gummy texture or crumb in the product. Hence, an accurate enzyme dosage at the outset is important. Enzymes already present in certain flours, syrups and other bakery ingredients also should be factored into the equation to determine overall enzyme activity.

In some cases, suppliers underestimate activity levels to compensate for the possible loss of activity during enzyme transport and warehousing. It is therefore important to determine whether there are any differences between the quoted ranges of activity and your laboratory-tested range of activity, therewith also saving substantially on production costs. Naturally, the more highly purified the enzyme is regarding its activity, the more expensive it will be. Understanding the limits of tolerance for side activities of a given enzyme can save costs, as well, by enabling to purchase the crudest, but also least expensive, enzyme that will work in the specific application.

Testing an enzyme is also advantageous to gauge any effects from equipment and production process.

In the art, CLEAs are prepared by precipitating the enzymes of interest by a precipitating agent, such as inorganic salts, e.g. ammonium sulphate, organic polymers, such as poly(ethyleneglycol) or organic solvents, such as tert-butylalcohol. However, the skilled person is aware of additional suitable precipitating agents, useable for aggregating the enzymes. The conditions for aggregating the enzymes are e.g. described in the art (Cao et al., Supra), and is common knowledge in the art. There are no restrictions for the enzymes to be used for immobilisation in the form of CLEAs, as long as the enzymatic activity is not hampered significantly by the aggregation and crosslinking process. For the skilled person, straight forward experimentations will give an indication about the suitability of the enzyme of interest to be immobilised by the CLEA technique. One of the advantageds of CLEAs is the fact that they can be prepared in the absence of solid carrier, although a carrier can be used if desired.

However, to obtain the CLEAs, the aggregated enzymes are crosslinked to one another by glutaraldehyde, a relatively short chained (α,ω) dialdehyde, having a backbone, connecting the α and ω termini, of only five carbon atoms.

Glutaraldehyde is known in the art for crosslinking proteins, such as enzymes, as the aldehyde groups are able to react with the amino residues of protein molecules, as well as with the primary amino groups of the e.g. lysine and arginine moieties in the peptide chain.

The present invention now provides a method for the preparation of CLEAs having improved enzymatic activity and is characterized in that the crosslinking agent is prepared by combining a first and a second compound each having at least two reactive groups, the reactive groups of the first compound being primary amino groups, the reactive groups of the second compound being aldehyde groups.

By such a combination, the primary amino groups of the first compound are able to react with an aldehyde group of the second compound, resulting in a reaction product, comprising the backbone of the first compound, linked, at the location of each of the original primary amino groups, to a second compound molecule, resulting in reaction products still having at least two functional aldehyde groups. In a thus prepared crosslinking agent, the aldehyde groups are spaced significantly further from one another compared with the distance of the aldehyde groups in glutaraldehyde. Without being bound to any explanation, it is thought that the use of the crosslinking agent according to the invention in the preparation of CLEAs results in the enzymes crosslinked to one another in a less tight spatial confirmation, i.e. providing more space between the crosslinked molecules, therewith enabling the said molecules to maintain its pre-determined spatial arrangement for proper enzymatic function. By crosslinking with a relatively short chained crosslinking agent, such as glutaraldehyde, the enzymes may be in such a close contact to one another that the optimal conformation for biocatalysis is hampered, leading to unproper folding and therewith to less enzymatic activity. Further, a greater distance between the enzymes may enable better accessibility of the substrate molecules to the active sites of the enzyme molecules.

By preparing CLEAs with a crosslinking agent according to the invention it is also possible to form CLEAs from enzymes that did not result in enzymatically active CLEAs when glutaraldehyde was used as crosslinking agent, probably due to sterical hinderance and hampered accessibility of the active sites of the said enzyme molecules, once crosslinked.

By varying the reaction conditions, e.g. the ratio between the first and second compound, temperature, pH, reaction time and concentration of the compounds, in the preparation of the crosslinking agent, polymeric forms of the crosslinking agent of variable modular length can be obtained, comprising a chain of alternating moieties of the first and second compound. Thus, the length of the backbone of the crosslinking agent can be optimised for the enzymes to be crosslinked in a very versatile manner by easy and straight forward experimentation.

Preferably, the first compound comprises at least two carbon atoms, the termini of the backbone being defined as α, ω respectively, the said termini both comprising the active groups. By using a first compound comprising two terminal primary amino groups, a crosslinking agent of unitary modular length can be obtained. However, compounds comprising more than two primary amino groups, or wherein the primary amino groups are not in the terminal α and ω position, can also be suitable for the preparation of the crosslinking agent according to the invention. Additional primary amino groups can be used to obtain branched crosslinking agent, although linear crosslinking agents are preferred in view of the above described unitary modular length of the crosslinking agent.

The first compound is preferably chosen from the group of compounds that have at least two primary amino groups. Preferably, the first compound is chosen from the group, consisting of:
i) diaminoalkanes such as ethylene diamine; 1,2- and 1,3-propane diamine; 1,4-butane diamine; 1,6-hexane diamine; 1, 8-octane diamine; 1,10-decane diamine; 1,11-undecane diamine; 1,12-dodecane diamines,
ii) multifunctional amines such as triamines such as tris (2-aminoethyl) amine,
iii) aromatic diamines such as Xylene diamines (p- or m- xylene diamines),
iv) diamines having at least one hetero atom between the amino groups such as 2-aminoethylether and, preferably, spermidin,
v) branched diamines such as 1,3-diamino-2-hydroxypropane.

The second compound preferably comprises a dialdehyde. However, compounds comprising more than two aldehyde groups can be suitable for the preparation of the crosslinking agent of the invention. Additional aldehyde groups can be used to obtain branched crosslinking agents, as discussed above. It is preferred for the second compound to comprise terminal aldehyde groups, i.e. in the α, ω configuration. Thus, in a special embodiment, the second compound comprises an (α, ω) dialdehyde. α, ω-Dialdehydes, the preparation and uses thereof are known in the art and described in e.g. US 4808756.

The said dialdehyde preferably comprises backbone of at least two carbon atoms, preferably an aliphatic backbone, preferably having at least a terminal dialdehyde group at both the α and ω termini of the said backbone. The carbon atoms of the aldehyde groups are considered to be part of the said backbone.

More preferably, the dialdehyde is chosen from the group, consisting of glutaraldehyde, glyoxal, 2,3-pentadione, 2,4-pentadione, 2,4-hexadione, 3,4-hexadione, 3-methyl-2,4-pentadione, 3-ethyl-2,4-pentadione, or a combination of two or more thereof. Most preferably, the second compound comprises glutaraldehyde. Glutaraldehyde is known to be a very potent crosslinking agent for peptides and proteins.

Regarding the above, it is prefered to prepare the crosslinking agent from an (α, ω) diamine and/or an (α, ω) dialdehyde. In order to produce linear crosslinking agents of controllable length, the first and second compound, each contain two reactive groups, the said reactive groups preferably being terminal groups. The length of such crosslinking agents can be varied in a modular way, i.e. by varying the ratio between the first and second compound, as is outlined above. The crosslinking agent of minimal length, herein defined as the "monomer", will consist of the molecular backbone of the first compound, such as an (α, ω) diamine, covalently linked at both termini thereof, to a second compound molecule. The next possible size of the crosslinking agent is formed by linking to the said molecule another diamine molecule (to one of the aldehyde groups of the "monomer") and by linkage of a diamine molecule to the second primary amino group of the said diamine. Thus, a modular length increase of the crosslinking agent can be obtained, the module consisting of one first compound molecule and one second compound molecule. It is however also possible to prepare a crosslinking agent according to the invention by using a mixture of a plurality of first and/or second compounds.

In an other preferred embodiment, the crosslinking agent is combined with an additional agent having crosslinking activity, for enzyme molecules. Such crosslinking agents are known in the art. Examples of such additional crosslinking agents are isocycanates, dihaloalkenes and terminal bisepoxides. The said additional crosslinking agents can be added to the crosslinking agent according to the invention before or at step C.

The crosslinking agent is preferably prepared in a substantially protein free environment, but can also be prepared in the presence of protein. It is advantageous to first prepare the crosslinking agent, and to crosslink the enzymes in a subsequent step.

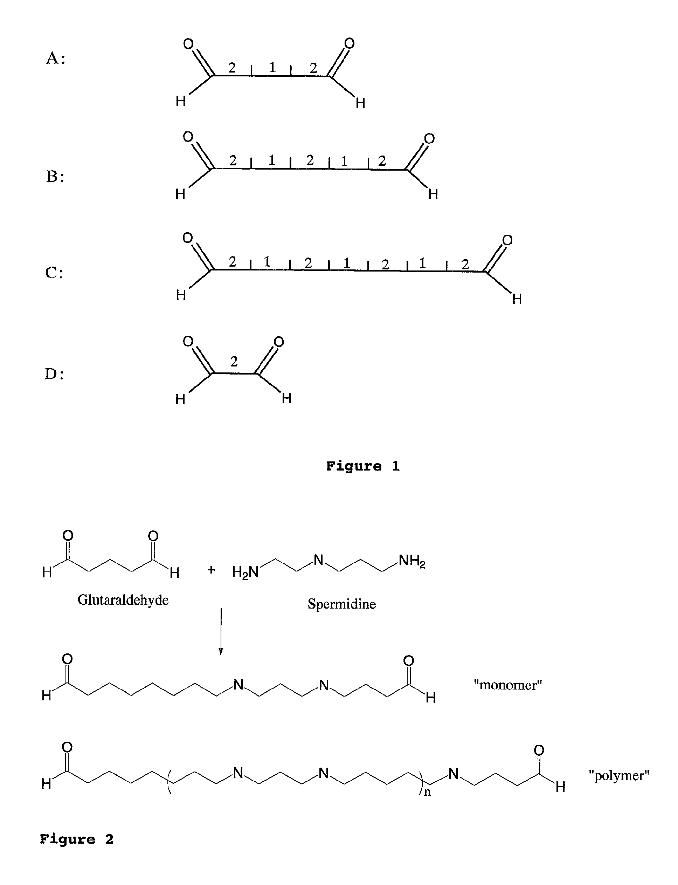

Preferably, the second and the first compound are combined in a molar ratio of 10-1:1, preferably 4-1:1, more preferably of 2,5-1,5:1, most preferably of 2:1. A molar excess of the first compound, i.e. having the reactive aldehyde groups, will result theoretically in the monomeric form of the crosslinking agent, i.e. of which the majority of the molecules consist of one molecule of the first compound, both primary amino groups thereof, being linked to a molecule of the second compound. Thus excess of the second compound is preferred to ensure the presence of the required free aldehyde groups on the crosslinking agent. The chemistry of the reaction between a dialdehyde, such as glutaraldehyde and a diamine is however rather complicated; in theory, when the ratio is about 1,5, the majority of the molecules of the crosslinking agent consists of two molecules of the first compound, linked together, through one of primary amino groups of each of the said molecules, by a molecule of the second compound. The free primary amino groups of both linked molecules of the first compound are each linked to a molecule of the first compound, resulting in a polymer having two reactive aldehyde groups. The agent thus formed contains one additional module as compared to the "monomer", as outlined above. In this way, by varying the ratio between the second and the first compound in the preparation of the crosslinking agent according to the invention in the range between 2 and 1, crosslinking compounds can be obtained, comprising a plurality of molecules of the first compound, linked together by molecules of the second compound. In order to avoid free primary amino groups, the ratio should preferably not equal 1 or being lower than 1. Free amino groups cannot participate in the envisaged protein crosslinking in the preparation of CLEAs. The preparation of crosslinking agent of different length is illustrated in figure 1. It is to be stressed that the above is a theoretical consideration; without being bound to any explanation, it is believed that the actual ratios are higher than the corresponding theoretical ratios.

Preferably, the crosslinking agent is prepared in an aqueous medium having a pH of 3 - 10, preferably in the range of 4 - 8, and a temperature of -5°C - 100°C, preferably of 10 - 40°C, wherein the concentration of the first and second compounds are in the range of 0,01 to 10 molar, preferably 0,3 - 3 molar. The reaction time is usually between 10s to 10hrs, preferably 1-2hrs. The invention also relates to crosslinked enzyme aggregates, obtainable by the invention.

The enzyme molecules are preferably chosen from lipases, esterases, proteases, nitrilases, oxinitrilases, penicillin amidases and amino acylases, the lipases preferably derived from Candida antarctica (form A), Candida antarctica (form B), Candida rugosa, Candida cylinracea, Porcine pancreatic, Candida lypolitica, Achromobacter spp., Penicillin Camenberti, Rhizopus Niveus, Aspergillus niger, Mucor javanicus, Alcaligenese spp., Pseudomonas cepacia, Pseudomonas fluorescens, Pseudomonas stutzeri, the esterases preferably derived from Candida Rugosa, Porcine liver, Rhizopus oryzee, Rhodotorula pilimenee, Bacillus stearothermophilus, Rhizomucor miehei, Streptomyces diastatochromogenes, Thermomyces lanuginosus, the proteases preferably derived from Aspergillus mellus, Aspergillus oryzea, Bacillus stearothermophillus, Rhizopus Niveas.

Further, the invention relates to a crosslinking agent, prepared by combining a first compound being an (α,ω) amine, preferably an (α,ω) diamine, preferably chosen from the group, consisting of diaminoalkanes, tris(2-aminoethyl)amine, 2 aminoethylether, ethylene diamine, xylene diamine, spermidine or a combination of two or more thereof, preferably spermidine and/or ethylene diamine with a dialdehyde, preferably chosen from the group consisting of glutaraldehyde, glyoxal, 2,3-pentadione, 2,4-pentadione, 2,4-hexadione, 3,4-hexadione, 3-methyl-2,4-pentadione, 3-ethyl-2,4-pentadione, or a combination of two or more thereof, the dialdehyde preferably being an (α,ω) dialdehyde, most preferably glutaraldehyde.

Further, the invention relates to the use of a crosslinking agent according to the invention for crosslinking a protein to another protein or to a carrier, preferably a solid carrier. In addition to the preparation of CLEAs, the said crosslinking agents have proven to be useful in a broad application of protein crosslinking reactions, as will be apparent to the persons skilled in the art.

The invention will further be illustrated by the figure and following examples.
Figure 1 schematically shows the preparation of the crosslinking agent according to the invention using different ratios of the first and second compound.
Figure 1a illustrates a crosslinking agent according to the present invention in monomeric form, i.e. obtainable when the ratio second compound:first compound is two or more. The molecular backbone of the crosslinking agent is depicted as a line; the free aldehyde groups are schematically shown at the ends thereof, i.e. at the α and ω position. The backbone is devided in 3 parts by two vertical lines indicating the position of the linkage of a second compound molecule (indicated by "(2)") to a first compound molecule (indicated by "(1)"). A crosslinking agent, one module larger in size is depicted in figure 1b. In comparison with the monomeric crosslinking agent in figure 1a, the crosslinking agent of figure 1b elongated by one module, comprises an additional first compound molecule and an additional second compound molecule. In figure 1c the next obtainable size of the crosslinking agent is shown. It is to be understood that the above is true when one kind of diamine is combined with one kind of a dialdehyde. As outlined above, a mixture of different diamines and/or aldehydes can be used to obtain a mixture of crosslinking agent molecules having increased variability in size. Figure 1d shows the situation when no compound 1 is present.
Figure 2 shows the preparation of an exemplary crosslinking agent according to the present invention, wherein spermidine as the first compound and glutaraldehyde as the second compound are used. The smallest crosslinking agent thus produced, the "monomer", consists of a molecular backbone of spermidine, at each of the amino termini thereof being linked to a glutaraldehyde molecule. Crosslinking agent molecules of larger size, so called "polymers" can be obtained by varying the ratio GAH:spermidine, the temperature, pH, reaction time and concentration as is indicated above. The module, consisting of a spermidine backbone, linked to a GAH backbone is indicated between brackets. "n" is an integer, indicating the number of modules present in the polymer.

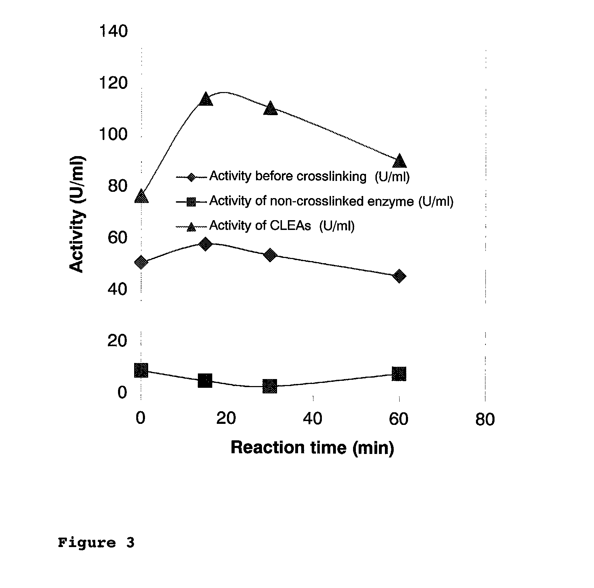

Figure 3 is a graph, illustrating the reaction time of glutaraldehyde with ethylene diamine on the residual activity of CLEAs of poreine sign pancreas lipase (PPL)of example 1.

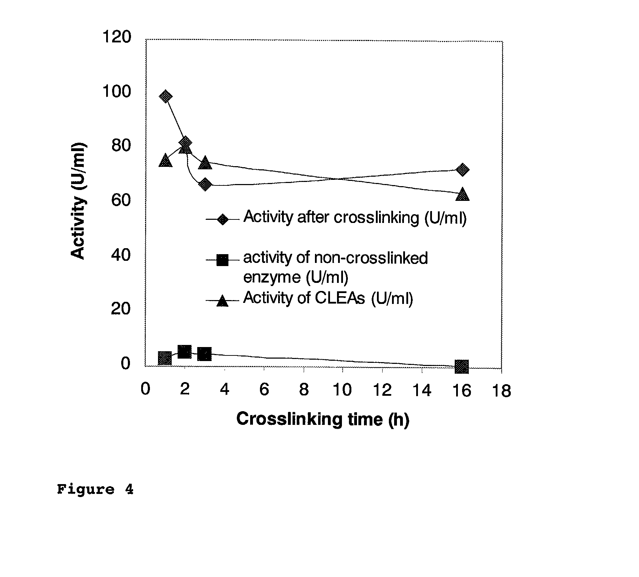

Figure 4 is a graph, illustrating the influence of crosslinking time on the residual activity of CLEAs of PPL according to example 2.

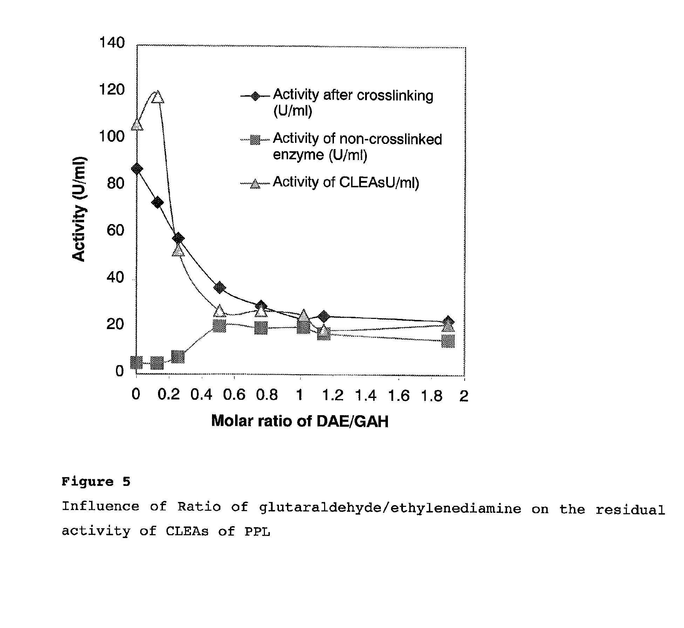

Figure 5 shows a graph of the influence of the ratio of glutaraldehyde:ethylene diamine on the residual activity of CLEAs according to example 3.

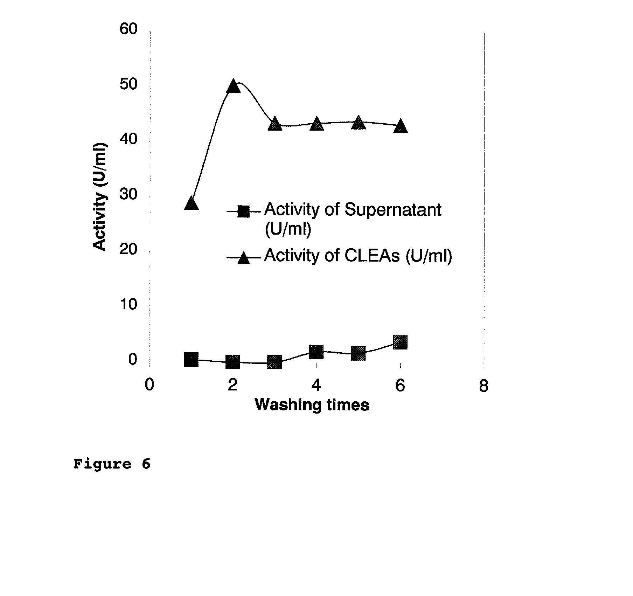

Figure 6 shows a graph, illustrating the effect of washing on the residual activity of CLEAs according to example 4.

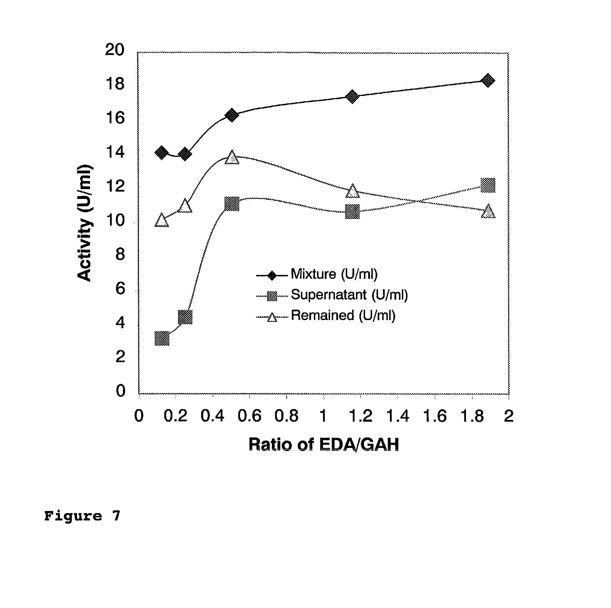

Figure 7 shows a graph, illustrating the influence of the ratio of ethylene diamine/glutaraldehyde for the preparation of CLEAs according to example 8.

### METHODS

### Assay of hydrolytic activity of lipases

The hydrolytic activity of various lipases was determined with a pH-stat (719 STAT Titrino, Metrohm, Schwitzerland). A certain amount of lipase preparation (solid or solution, immobilized or free lipase) was added to 50 ml phosphate buffer, 0.1 M, pH8 (thermostated at 37°C) containing 3 ml triacetin. The solution was automatically titrated with NaOH solution (0.1M) to the desired pH before the lipase was added. The consumption of NaOH was recorded as function of time. The lipase activity was then expressed in Units (U). One unit corresponds to the liberation of 1 µmol fatty acid per minute.

### Assay of hydrolytic activity of penicillin G acylase

The activity of the samples was measured by adding a definite volume or weight to 25 ml 100 mM phosphate buffer, pH 8.0 containing 2% (w/v) fresh penicillin G. During the assay, the solution was thermostated at 25°C. The solution was automatically titrated with 0.1 M NaOH using a 718 pH stat (Metrohm, Switzerland. One unit U of penicillin acylase will liberate 1 µmol of phenylacetic acid per min.

### HPLC-analysis

All synthetic reaction mixtures were analyzed by an HPLC system equipped with an HPLC system using a chiral column (OD) (UV-detector at 215 nm and a Spectra Physics SP 4400 integrator; mobile phase n-hexane: iso-propanol 97:3 at a flow rate of 0.5 ml/min at 25 °C.

### Example 1:

### Influence of time for the preparation of novel crosslinkers

### 1.1 Preparation of enzyme solution

2.5 gram Hog pancreas lipase from Fluka was added in 0.2 M potassium buffer and stirred at room temperature for 30 minutes. The un-dissolved components were removed by centrifugation. The clear enzyme solution so prepared is ready for following aggregation.

### 1.2 Preparation of enzyme aggregates

To above mentioned clear enzyme solution 5 ml, 2 gram PEG 6000 was slowly added under stirring at 0°C (in ice-water-bath). The mixture was left stirring at 0°c for 30 minutes.

### 1.3 Preparation of crosslinker

100 µl EDA (0.33M, pH6.0, adjusted with 6 N HC1 aqueous solution) was mixed with 100µl 25% glutaraldehyde solution (2.6 M) and stirred at room temperature for the certain time as specified in Table 1 (0 minutes, 15 minutes, 30 minutes and 60 minutes) and was added to the above prepared enzyme aggregates solution.

### 1.4 Crosslinking enzyme aggregates

After addition of the above-prepared crosslinker, the mixture was stirred at 0°C in an ice-water bath for 2 hours. The activity of the reaction mixture was measured according to the method described in the above method section. After centrifugation, the solid CLEAs were dispersed in 0.1 M potassium phosphate buffer, pH 7.8 to the original volume by vortexing. Again, the activity in the supernatant, namely the non-crosslinked enzyme (see Table 1, column 3) was measured. The activity of remaining solid CLEAs was measured by addition of the same buffer used above to the original volume and vortexing.

### 1.5 Results

It was found that the optimal mixing time for preparation of the crosslinker is about 20 minutes (see Figure 1). Direct addition of the two components, namely ethylene diamine and glutaraldehyde resulted in a CLEA with 30% lower activity ( see Table 1), indicating that the a certain mixing time is preferred to allow the growth of the crosslinker length.

**Table 1**

| Influence of reaction time for the preparation of novel crosslinker on the residual activity of CLEAs of PPL | | | |
|---|---|---|---|
| Reaction time (Minute) | Activity after crosslinking ^{a} (U/ml) | Activity of non-crosslinked enzyme (U/ml)* | Activity of CLEAs ^{b} (U/ml)* |
| 0 | 51.5 | 9.6 | 77.3 |
| 15 | 58.6 | 5.7 | 115 |
| 30 | 54.5 | 3.6 | 111.6 |
| 60 | 46.6 | 8.7 | 91.4 |

| | | | |
|---|---|---|---|
| * After washing; ^{a} after crosslinking; ^{b} After centrifugation and adding buffer to the original volume of mixture; ^{c}500 µl Crosslinker | | | |

### Example 2:

### Influence of Crosslinking time on the residual activity of CLEAs of PPL using crosslinker prepared with ethylene diamine: glutaraldehyde (1:1 v/v)

### 2.1 Preparation of enzyme solution

2.5 gram Hog pancreas lipase from Fluka was added in 0.2 M potassium buffer and stirred at room temperature for 30 minutes. The un-dissolved components were removed by centrifugation. The clear enzyme solution so prepared is ready for following aggregation.

### 2.2 Preparation of enzyme aggregates

To above-mentioned clear enzyme solution 5 ml, 2 gram PEG 6000 was slowly added under stirring at 0°C (in ice-water-bath). The mixture was left stirring at 0°C for 30 minutes.

### 2.3 Preparation of crosslinker

100 µl EDA (0.33M, pH6.0, adjusted with 6 N HCl aqueous solution) was mixed with 100µl 25% glutaraldehyde solution (2.6 M) and stirred at room temperature for 5 minutes and was added to the above prepared enzyme aggregates solution to initialize the crosslinking.

### 2.4 Crosslinking enzyme aggregates

After addition of the above-prepared crosslinker, the mixture was stirred at 0°C in an icewater bath for the time as specified in Table 2. The activity of the reaction mixture was measured according to the method described in the above method section. After centrifugation, the solid CLEAs were dispersed in 0.1 M potassium phosphate buffer, pH 7.8 to the original volume by vortexing. Again, the activity in the supernatant, namely the non-crosslinked enzyme (see Table 2, column 3) was measured. The activity of remaining solid CLEAs was measured by addition of the same buffer used above to the original volume and vortexing.

### 2.5 Results and discussion

It has been found that the residual activity after crosslinking is usually decreased as the crosslinking time increased (see table 2, column 2). The highest activity of CLEAs was obtained after 2-hour crosslinking. Although higher residual activity can be obtained after 1-hour crosslinking (see Table 2 column 2), it gave a CLEAs with moderate activity retention, due to the fact that not all enzyme aggregates are crosslinked in short time.

**Table 2:**

| Influence of Crosslinking time on the residual activity of CLEAs of PPL using crosslinker prepared with ethylene diamine: glutaraldehyde (1:1 v/v) | | | |
|---|---|---|---|
| Crosslinking time (h) | Activity after crosslinking (U/ml) | Activity of non-crosslinked enzyme (U/ml) | Activity of CLEAs (U/ml) |
| 1 | 99.2 | 3 | 75.6 |
| 2 | 82.2 | 5.3 | 80.5 |
| 3 | 66.8 | 4.5 | 75 |
| 16 | 73.1 | 0.23 | 64 |

### Example 3:

### Influence of ratio of glutaraldehyde/ethylenediamine on the residual activity of CLEAs of PPL

### 3.1 Preparation of enzyme solution

2.5 gram Hog pancreas lipase from Fluka was added in 0.2 M potassium buffer and stirred at room temperature for 30 minutes. The un-dissolved components were removed by centrifugation. The clear enzyme solution so prepared is ready for following aggregation.

### 3.2 Preparation of enzyme aggregates

To above mentioned clear enzyme solution 5 ml, 2 gram PEG 6000 was slowly added under stirring at 0°C (in icewater-bath). The mixture was left stirring at 0°c for 30 minutes.

### 3.3 Preparation of crosslinker

Ethylene diamine solution (0.33 M, pH 6.0, adjusted with 6 N HCl aqueous solution) was mixed with 25% glutaraldehyde aqueous solution (2.6 M) according to the molar ratio specified in Table 3 and stirred at room temperature for 5 minutes. 200 µl above prepared crosslinkers were respectively added to the above prepared enzyme aggregates solution to initialize the crosslinking.

### 3.4 Crosslinking enzyme aggregates

After addition of the above-prepared crosslinker, the mixture was stirred at 0°C in an icewater bath for certain time as specified in Table 2. The activity of the reaction mixture was measured according to the method described in the method section of this patent application. After centrifugation, the solid CLEAs were dispersed in 0.1 M potassium phosphate buffer, pH 7.8 to the original volume by vortexing. Again, the activity in the supernatant, namely the non-crosslinked enzyme (see Table 2, column 3) was measured. The activity of remaining solid CLEAs was measured by addition of the same buffer used above to the original volume and vortexing.

### 3.5 Results and discussion

It has been found in the experiment that the optimal volume ratio of ethylene diamine (0.33 M)/glutaraldehyde (2.6 M) was 1:1 (i.e. a molar ratio of 7.9). Besides, it was found that the remaining activity of CLEAs (see Table 3, column 4) generally decreased, as the ratio of ethylene/glutaraldehyde increased. The reason for the lower activity retention of CLEAs is ascribed to the presence of excess diamines that probably result in the formation of compounds with less aldehyde functionalities for crosslinking. In another words, it is believed that not all the enzyme aggregates are crosslinked together in the case of higher molar ratio of ethylene diamine/glutaraldehyde.

**Table 3:**

| Influence of Ratio of glutaraldehyde/ethylene diamine on the residual activity of CLEAs of PPL | | | |
|---|---|---|---|
| Molar Ratio of ethylene diamines to glutaraldehyde | Activity after crosslinkin g (U/ml) | Activity of non-crosslinked enzyme (U/ml) | Activity of CLEAs (U/ml) |
| 0:1 | 87.4 | 4.6 | 106.3 |
| 1:7.88 | 73.0 | 4.5 | 118.1 |
| 1:3.94 | 57.8 | 7.2 | 52.9 |
| 1:1.97 | 36.9 | 20.6 | 27.1 |
| 1:1.31 | 29.1 | 19.7 | 27.2 |
| 1:0.985 | 23.6 | 20.0 | 25.3 |
| 1:0.875 | 24.9 | 17.5 | 19.0 |
| 1:0.525 | 23.0 | 14.7 | 21.5 |

### Example 4:

### Enzyme leakage experiments of CLEAs of PPL

### 4.1 Preparation of enzyme solution

2 gram Hog (porcine) pancreas lipase from Fluka was added in 25 ml 0.2 M potassium buffer, pH7.5 and stirred at room temperature for 30 minutes. The un-dissolved components were removed by centrifugation. The clear enzyme solution so prepared is ready for following aggregation.

### 4.2 Preparation of enzyme aggregates

To above mentioned clear enzyme solution 5 ml, 2 gram PEG 6000 was slowly added under stirring at 0°C (in ice-water-bath). The mixture was left stirring at 0°C for 30 minutes.

### 4.3 Preparation of crosslinker

Ethylene diamine aqueous solution (0.33 M, pH 6.0, adjusted with 6 N HCl) was mixed with 500 µl 25% glutaraldehyde aqueous solution (2.6 M) and stirred at room temperature for 5 minutes. 200 µl above prepared crosslinker was added to the above prepared enzyme aggregates solution to initialize the crosslinking.

### 4.4 Crosslinking enzyme aggregates

After addition of the above-prepared crosslinker, the mixture was stirred at 0°C in an icewater bath for 2 hours. 1.5 ml of the mixture was taken from the above mixture and was centrifuged. The supernatant was poured out and 0.2M potassium phosphate buffer, pH 7.5 was added to reach 1.5 ml. The solution was mixed and then centrifuged again. The hydrolytic activity of the supernatant was measured using the method described in this patent. The remaining CLEAs was suspended/dispersed again and the mixture was measured for its hydrolytic activity. During each washing step, the supernatant was removed by centrifugation and buffer was added to reach the same volume

### 4.5 Results and discussion

As shown in Table 1 and 2, the residual activity of CLEAs of PPL was generally increased after washing with buffer. In order to establish whether the times for washing can effect the residual activity, the same sample of CLEAs of PPL after crosslinking was divided into several equal portions and washed for different times as indicated in Figure 1.

It was found that more washing times has no influence on the residual activity (as shown in Figure 1), indicating that all the non-crosslinked enzyme has been washed away in the first washing step. Moreover, it has been found that the activity of CLEAs is not decreased after 5 times washing providing the evidence that no leakage was found with CLEAs of PPL in aqueous media.

### Example 5:

### Influence of different Crosslinkers prepared by Glutaraldehyde and different compounds with diamine functionalities on the residual activity of CLEAs of PPL

### 5.1 Preparation of enzyme solution

2 gram Hog (porcine) pancreas lipase from Fluka was added in 20 ml 0.2 M potassium buffer, pH 7.5 and stirred at room temperature for 30 minutes. The un-dissolved components were removed by centrifugation. The clear enzyme solution so prepared is ready for following aggregation.

### 5.2 Preparation of enzyme aggregates

To above mentioned clear enzyme solution 5 ml, 2 gram PEG 6000 was slowly added under stirring at 0°C (in icewater-bath). The mixture was left stirring at 0°C for 30 minutes.

### 5.3 Preparation of crosslinkers

1 ml diamine aqueous solution (0.33M, pH6.0, adjusted with 6 N HCl) as specified in Table 5 was mixed with 500 µl 25% glutaraldehyde aqueous solution (2.6 M) and stirred at room temperature for 5 minutes. 500 µl above prepared crosslinker was added to the above prepared enzyme aggregates solution to initialize the crosslinking.

### 5.4 Crosslinking enzyme aggregates

After addition of the above-prepared crosslinker, the mixture was stirred at 0°C in an icewater bath for 2 hours. The activity of the reaction mixture was measured according to the method described in the above method section. After centrifugation, the solid CLEAs were dispersed in 0.1 M potassium phosphate buffer, pH 7.8 to the original volume by vortexing. Again, the activity in the supernatant, namely the non-crosslinked enzyme (see Table 5, column 3) was measured. The activity of remaining solid CLEAs was measured by addition of the same buffer used above to the original volume and vortexing.

### 5.5 Results and discussion

As shown in Table 5, variation of the diamine compound for the preparation of glutaraldehyde derivatives as multifunctional crosslinkers generally leads to the formation of CLEAs with higher activity, when compared with glutaraldehyde. Besides, it is possible to modulate the properties of the CLEAs by selecting different diamine compounds.

**Table 5**

| Influence of different Crosslinkers prepared by Glutaraldehyde and different compounds with diamines functionalities on the residual activity of PPL | | | | |
|---|---|---|---|---|
| Crosslinker | Activity after crosslinking (U/ml) | Activity of non-crosslinked enzyme (U/ml)* | Activity of CLEAs (U/ml)* | Relative activity^{†} |
| GAH | 65.0 | 5.3 | 85.9 | 1.00 |
| TAEA | 60.3 | 11.1 | 113 | 1.32 |
| EDA | 73.3 | 10.7 | 118.6 | 1.38 |
| m-XDA | 80.7 | 7.3 | 92.8 | 1.08 |
| 2-AEE | 88.6 | 11.6 | 114.7 | 1.34 |

| | | | | |
|---|---|---|---|---|
| *After washing; | | | | |
| ^{†} with the residual activity of CLEAs prepared with only glutaraldehyde as 1 GAH: Glutaraldehyde EDA: Ethylene diamine m-XDA: m-Xylenediamine TAEA: Tris (2-aminoethyl) amine, 2-AEE: 2-aminoethylether; | | | | |

### Example 6:

### Comparison of crosslinking enzyme aggregates of PGA using glutaraldehyde and crosslinker prepared by glutaraldehyde and spermidine

### 6.1 Preparation of enzyme solution

5 ml penicillin G acylase aqueous solution from Fluka was diluted in 5 ml enzyme in 5 ml 0.2 M potassium phosphate buffer, pH 7.5.

### 6.2 Preparation of enzyme aggregates

To 5 ml the above enzyme solution, PEG 6000 was slowly added to 40% (w/v) at 0°C in a water-ice bath. The mixture was left stirring 0°C for 1 hour. No activity was found in the supernatant after 1-hour precipitation.

### 6.3 Preparation of the crosslinker

200 µl spermidine solution (0.33M, pH6.0, adjusted with 6 N HCl was mixed with 200 µl 25% glutaraldehyde aqueous solution (2.6 M) and stirred at room temperature for 5 minutes.

### 6.4 Crosslinking enzyme aggregates of penicillin G acylase

200 µl glutaraldehyde or 200 µl above-prepared crosslinker were respectively added to the above prepared enzyme aggregates solution to initialize the crosslinking. The mixture was stirred at 0°C in a water-ice bath for 2 hours. After the crosslinking, the CLEAs were collected by centrifugation. New buffer was added to wash the non-crosslinked enzyme. After wards, the activity of the remaining CLEAs and the supernatant containing non-crosslinked enzyme was measured.

### 6.5 Results

As shown in Table 6, high activity retention was obtained with the new crosslinker prepared with glutaraldehyde and spermidine.

**Table 6**

| Comparison of crosslinking enzyme aggregates of PGA using glutaraldehyde and crosslinker prepared by glutaraldehyde and spermidine | | | | |
|---|---|---|---|---|
| Crosslinker | Activity input | Activity of CLEAs of PGA (U) | Activity of non-crosslinked PGA (U) | Residual activity of CLEA (%) |
| GAH | 1530 | 1341 | 16.2 | 87.6 |
| GAH+SM | 1530 | 1587 | 6.6 | 103.7 |
| GAH: Glutaraldehyde | | | | |
| SM: spermidine | | | | |

### Example 7:

### Influence of variation of the chain length of diamines on the preparation of crosslinked enzyme aggregates

### 7.1 Preparation of CLEAs of penicillin G acylase

Penicillin G acylase (purchased from Fluka) solution was 5 times diluted with 0.2 M potassium phosphate buffer, pH 8.0.

To 3 ml above enzyme solution, 6 ml *tert*-butanol was added at 0°C under stirring for 1 hour. Then, 250 µl crosslinker prepared by mixing glutaraldehyde (2.6 M) with the desired diamine solution (0.33 M, pH6.0), as specified in Table 7, was added. After 1 hour crosslinking at 0°C, the activity of the mixture was measured. After centrifugation, the supernatant was discarded and new buffer was added. After washing the CLEAs (prepared with each crosslinker) with 0.1 M potassium phosphate buffer, pH7.5, the remaining activity of CLEAs was measured.

### 7.2 Preparation of CLEAs of Candida rugosa lipase (CRL)

3 g Candida rugosa purchased from Fluka was dissolved in 30 ml potassium phosphate buffer pH 7.5 (0.2M) and centrifuged to remove the un-dissolved components; Aggregation conditions: to 4 ml above enzyme solution, 2 g PEG 6000 was added slowly under stirring at 0°C to reach 50% (w/v);

The mixture was stirred at 0°C for 1 hour in an ice bath; Afterwards, different crosslinker 500µl that was prepared by mixing 250 µl diamine (0.33 M, pH 6.5 adjusted with 6 N HCl) with 250 µl glutaraldehyde solution (2.6 M) for 5 min at room temperature was added to the corresponding mixture to initialize the crosslinking. The crosslinking was performed at 0°C for 1 hour under stirring

### 7.3 Preparation of CLEAs of Candida antaractica lipase B (CAL-B)

To 5 ml lipase sp525 (*Candida antaractica* lipase) solution dispersed in 0.1 M, pH7.5, potassium phosphate buffer), 2.5 g PEG 6000 was slowly added to reach 50% (w/v) under stirring at 0°C. The mixture was stirring at 0°C for 1 hour. Then, 400 ml glutaraldehyde or the glutaraldehyde derivatives prepared by mixing 200 µl 0.33 M diamine specified in the table (pH 6.0, adjusted with 6 N HCl) and 200 µl 2.6 M glutaraldehyde at RT for 5 minutes. Crosslinking was performed at 0°C for 1 hour and 16 hour at 4°C. After the crosslinking, CLEAs formed were isolated by centrifugation and washing with 0.1 M and filtered to remove the water until no water drops come out.

### Results

The results were surmised in Table 7.1. It was found that the residual activity of CLEAs was generally increased by using glutaraldehyde derivatives with diamines, compared with only glutaraldehyde as crosslinker.

The activity of CLEAs of some enzymes can be increased up to 100%.

The reason for the activity increase might be attributed to the change of CLEAs surface properties by using different crosslinkers.

The optimal chain length is probably dependent on the properties of each enzyme.

**Table 7.1**

| Influence of chain length of diamine compounds on the residual activity of CLEAs of Candida rugosa lipase (CRL) | | | |
|---|---|---|---|
| CLEAs | Crosslinker | Activity retention after crosslinking ^{a}(%) | Relative activity ^{b} |
| | **GAH** | 68 | 1 |
| | GAH-DAE | 51 | 1.473 |
| PGA | GAH-DAB | 59.6 | 1.38 |
| | GAH-DAO | 22.2 | 0.39 |
| | GAH-DAD | 56.6 | 1.24 |
| | **GAH** | 48 | 1.0 |
| | GAH+DEA | 70 | 1.46 |
| CRL | GAH+ DAB | 86 | 1.79 |
| | GAH+ DAO | 86 | 1.79 |
| | GAH + DAD | 96 | 2 |
| CAL-B | GAH | 98 | 1.0 |
| | GAH+DEA | 139 | 1.4 |
| | GAH+ DAB | 114 | 1.2 |
| | GAH+ DAO | 109 | 1.1 |
| | GAH + DAD | 149 | 1.5 |

| | | | |
|---|---|---|---|
| ^{a}Activity retention is defined as : total activity used for preparation of CLEA/ total activity of the CLEAs; ^{b} with CLEAs prepared with glutaraldehyde as 1. PGA: Penicillin G acylase; CRL: Candida rugosa lipase GAH: Glutaraldehyde. DAD: 1, 12 diamino decane; DAB: 1, 4-diaminobutane; DAO: 1, 8, Diaminooctane; DEA: Diaminoethane | | | |

### Example 8:

### Influence of ratio of ethylenediamine /glutaraldehyde for the preparation of CLEAs of Candida rugosa lipase (CRL)

### 8.1 Preparation of enzyme solution

3 g *Candida rugosa* lipase purchased from Fluka was dissolved in 30 ml 0.2 M potassium phosphate buffer, pH 7.5. and stirred at room temperature for 30 minutes. The un-dissolved components were removed by centrifugation. The clear enzyme solution so prepared is ready for following aggregation.

### 8.2 Preparation of enzyme aggregates

To 5 ml above enzyme solution, solid PEG 6000 was added to reach 40% (w/v) under stirring at 0°C (in ice-water-bath). The mixture was left stirring at 0°C for 30 minutes.

### 8.3 Preparation of crosslinker

0.33 M ethylene diamine aqueous solution (pH 6.5 adjusted with 6 N HCl) was mixed with 25% glutaraldehyde aqueous solution (2.6 M) under stirring for 5 min at room temperature. The molar ratio of ethylene diamine to glutaraldehyde was listed in Table 8.

### 8.4 Crosslinking enzyme aggregates

200 µl crosslinker prepared by varying the molar ratio of ethylene diamine to glutaraldehyde was added respectively to each reaction mixture. The mixture was stirred at 0°C in ice-water bath for 1 hour.

After crosslinking, 2 ml mixture was transferred into an Eppendorf and centrifuged for 5 min at 13000 rpm. After centrifugation the supernatant was removed and the residual solid was diluted wit 2 ml 0.1 M potassium phosphate buffer, pH 7.8. This mixture was vortexed until CLEAs was completely dispersed. After centrifugation, the activity of the supernatant was measured.

After the activity measurement, the enzyme was dispersed in the same buffer solution, and the activity of the mixture was measured.

### 8.5 Results

As the molar ratio of ethylene diamine /glutaraldehyde increased, it was found that the remaining activity after crosslinking increased (see Table 8, column 2). At the meantime, the activity of the non-crosslinking enzyme activity increased. The optimal molar ratio of the two components can be variable, depending on the properties of the enzyme and chemical properties of the diamine compound.

**Table 8**

| Influence of ratio of ethylene diamine /glutaraldehyde for the preparation of CLEAs of Candida rugosa lipase | | | |
|---|---|---|---|
| Molar ratio of EDA/GAH | Activity of mixture after crosslinking (U/ml) | Activity of non-crosslinked enzyme (U/ml) | Activity of CLEAs (U/ml) |
| 1:7.88 | 14.12 | 3.24 | 10.2 |
| 1:3.94 | 14.04 | 4.48 | 11.04 |
| 1:1.97 | 16.32 | 11.12 | 13.88 |
| 1:0.95 | 17.44 | 10.68 | 11.92 |
| 1:0.53 | 18.4 | 12.24 | 10.76 |
| EDA: ethylene diamine | | | |
| GAH: Glutaraldehyde | | | |

### Example 9

### Comparison experiments of different enzyme preparations of CAL-B

### 9.1 Hydrolytic activity of different preparations of CAL-B

The different enzyme preparations of CAL-B such as CLEA of CAL-B, Sol-gel-CAL-B, CLEC-CAB (from Altus), L-2 (Roche) and native enzyme (lyophilized Cal-B from Roche) were used to measure their hydrolytic activity in aqueous medium (see Method).

### 9.3 Transesterification activity of different preparations of CAL-B in organic solvents

To determine the enzyme activity in organic solvents, a batch reactor thermostated at 20°C in was used. To 10 ml organic toluene containing 10 mmol racemic 1-phenyl ethanol and 10 mmol vinyl acetate was solid lipase preparation added.

The reaction mixture was gently stirred at 20°C. 5µl Sample were taken at interval of 15 min and dissolved in 1ml iso-propanol 97:3 (v /v) and centrifuged at 13000 rpm for 5 minutes prior to analysis.

One unit (U) of synthetic activity of the solid lipase preparations corresponds to the formation of 1 µmol of R-1-phenyl ethyl acetate per min.

### 9.4 Results

As shown in Table 9, CLEAs of CAL-B is almost 40 times active, compared with the commercial preparation i.e. L-2 immobilised on the carrier. CLEC-CAL-B was prepared from the purified enzyme. Hence, it is not surprising that CLEC-CAB has the highest specific hydrolytic activity in aqueous medium. In contrast, the hydrolytic activity of CAL-B is only half of CLEC-Cal-B, because it was prepared from the crude enzyme without any purification.

As shown in Table 9, CLEA-CAL-B has the highest activity for transesterification in organic solvent, while the CLEC-CAL-B displayed a marginal transesterification activity in organic solvent, indicating that CLEC-CAL-B was essentially not active in pure organic solvent.

**Table 9**

| Comparison of CLEAs of CAL-B (Candida antaractica lipase B) with CLEC-CAB and sol-gel-CAL-B | | | |
|---|---|---|---|
| Enzyme Candida antaractica Lipase B | Specific activity ^{hydr} (KU/g dry)^{e} | Specific activity ^{Syn} (KU/g)^{f} | S/H^{g} |
| CLEA* | 18.6 | 8.19* | 0.44 |
| Sol gel | 3.9 | 1.21 ^{a} | 0.31 |
| CLEC | 42.1 | 1.26 ^{b} | 0.03 |
| L-2 (c-f) ^{c} | 0.44 | 0.5 | <1.2 |
| Native enzyme^{d} | 9.7 | 4.50 | 0.47 |

| | | | |
|---|---|---|---|
| * CLEAs of CAL-B was prepared using the derivative of glutaraldehyde with ethylene diamines according to the method described in example 7.3 | | | |
| ^{a} about 33% protein content; * water content less than 20% | | | |
| ^{b} about 20% water; | | | |
| ^{c}c-f Carrier fixed commercialized immobilized lipase Candida antaractica lipase B (Roche) | | | |
| ^{d} Lyophilized enzyme powder | | | |
| ^{e} hydrolytic activity is based on the hydrolysis of glycerol triacetate. One unit is defined as the amount of enzyme required to release 1 mmol acid/minute; | | | |
| ^{f} Synthetic activity is measured as the initial reaction rate in the transesterification between 1-phenylethanol and vinyl acetate; | | | |
| ^{g} S / H c is an important parameter for the evaluation of how active the immobilized enzyme is in organic solvent relative to the aqueous medium. | | | |

## Claims

1. Method for the preparation of crosslinked enzyme aggregates, comprising the steps of:
A - providing a plurality of enzyme molecules,
B - aggregating the enzymes in a liquid medium, comprising a precipitating agent,
C - crosslinking the aggregated enzymes to one another by providing a crosslinking agent in the liquid medium,
**characterized in that** the crosslinking agent being prepared by combining a first and a second compound each having at least two reactive groups, the reactive groups of the first compound being primary amino groups, the reactive groups of the second compound being aldehyde groups.

2. Method according to claim 1, wherein the first compound comprises at least two carbon atoms, the termini of the backbone being defined as α and ω, respectively, the said termini both comprising the active groups.

3. Method according to claim 1 or 2, wherein the first compound is chosen from the group consisting of diaminoalkanes, triamines, aromatic diamines, diamines having at least one hetero atom between the amino groups, branched diamines or a combination of two or more thereof.

4. Method according to any of the preceding claims, wherein the second compound is a dialdehyde.

5. Method according to any of the preceding claims, wherein the second compound is chosen from the group, consisting of glutaraldehyde, glyoxal, 2,3-pentadione, 2,4-pentadione, 2,4-hexadione, 3,4-hexadione, 3-methyl-2,4-pentadione, 3-ethyl-2,4-pentadione, or a combination of two or more thereof.

6. Method according to any of the preceding claims, wherein the crosslinking agent is prepared in a substantially protein free environment.

7. Method according to any of the preceding claims, wherein the second and the first compound are combined in a molar ratio of 10-1:1, preferably 4-1:1, more preferably of 2,5-1,5:1, most preferably of 2:1.

8. Method according to any of the preceding claims, wherein the enzyme molecules are chosen from lipases, esterases, proteases, nitrilases, oxynitrilases, penicillin amidases and amino acylases.

9. Crosslinked enzymes aggregate, obtainable by any of the preceding claims.

10. Crosslinking agent prepared by combining a first compound as defined in any of the claims 2-3 with a second compound as defined in any of the claims 4-5.

11. Use of a crosslinking agent according to claim 10 for crosslinking a protein to another protein or a carrier, preferably a solid carrier.
